# EUROPEAN PATENT SPECIFICATION

(11) **EP 3 934 739 B1**
(45) Date of publication and mention of the grant of the patent: **04.09.2024**
(21) Application number: 20716278.5
(22) Date of filing: 05.03.2020
(51) Int. Cl.: A61N 1/36, A61B 5/00, A61B 5/11, A61B 5/296, A61B 5/389, A61N 1/05, A61B 7/00, A61B 7/02, G16H 40/63, G16H 50/20, A61N 1/375, A61B 5/024, A61B 5/394

(54) **LINGUAL MUSCLE TONE SENSING SYSTEM FOR IMPROVED THERAPY OF OBSTRUCTIVE SLEEP APNEA**
LINGUALMUSKELTONUSMESSSYSTEM FÜR VERBESSERTE THERAPIE DER OBSTRUKTIVEN SCHLAFAPNOE
SYSTÈME DE DÉTECTION DE TONUS MUSCULAIRE LINGUAL DE THÉRAPIE DE L'APNÉE OBSTRUCTIVE DU SOMMEIL

(30) Priority: 06.03.2019 US 201962814398 P; 24.01.2020 US 202016752363
(43) Date of publication of application: 12.01.2022
(73) Proprietor: Medtronic Xomed, LLC, Jacksonville, FL 32216 (US)
(72) Inventor: SCHEINER, Avram, Mounds View, Minnesota 55112 (US); SCHULHAUSER, Randal, Tempe, Arizona 85281-5133 (US); HISSONG, James, Jacksonville, Florida 32216 (US); SCOTT, Erik, Minneapolis, Minnesota 55432 (US); HAAG, Rebecca, Boulder, Colorado 80301 (US)
(74) Representative: Dehns
(86) International application number: PCT/US2020/021246
(87) International publication number: WO 2020/181124

(56) References cited:
- US-A1- 2007 150 006
- US-A1- 2017 197 075

## Description

### TECHNICAL FIELD

This disclosure relates to a medical device system and method for therapeutic electrical stimulation of the hypoglossal nerve for treatment of obstructive sleep apnea. More particularly the disclosure relates to methods of measuring lingual muscle tone and evaluating efficacy of stimulation therapy.

### BACKGROUND

Implantable medical devices capable of delivering electrical stimulation pulses have been proposed or are available for treating a variety of medical conditions, such as cardiac arrhythmias and chronic pain as examples. Obstructive sleep apnea (OSA), which encompasses apnea and hypopnea, is a serious disorder in which breathing is irregularly and repeatedly stopped and started during sleep, resulting in disrupted sleep and reducing blood oxygen levels. OSA is caused by complete or partial collapse of the pharynx during sleep. In particular, muscles in a patient's mouth and throat intermittently relax thereby obstructing the upper airway while sleeping. Airflow into the upper airway can be obstructed by the tongue or soft pallet moving to the back of the throat and covering a smaller than normal airway. Loss of air flow also causes unusual inter-thoracic pressure as a person tries to breathe with a blocked airway. Lack of adequate levels of oxygen during sleep can contribute to abnormal heart rhythms, heart attack, heart failure, high blood pressure, stroke, memory problems and increased accidents. Additionally, loss of sleep occurs when a person is awakened during an apneic episode. Implantable medical devices capable of delivering electrical stimulation pulses have been proposed for treating OSA by electrically stimulating muscles around the upper airway that may block the airway during sleep.

### SUMMARY

The invention provides an implantable neurostimulator according to claim 1. For better understanding of the invention, the present disclosure provides also further, non-claimed, aspects, which do not form part of the invention but are provided for illustrative purposes. One aspect of the disclosure is directed to an implantable neurostimulator (INS) including: an electrical lead having formed thereon at least a pair of bi-polar electrodes, where the electrical lead is configured for placement of the pair of bi-polar electrodes proximate protrusor muscles of a patient and configured to receive electromyography (EMG) signals; a pulse generator electrically connected to the electrical lead and configured to deliver electrical energy to the pair of bi-polar electrodes, the pulse generator having therein a sensor and a control circuit, where the sensor and control circuit are configured to receive the EMG signals and determine a tonal state of the protrusor muscles in which the lead is placed. Other embodiments of this aspect include corresponding computer systems, apparatus, and computer programs recorded on one or more computer storage devices, each configured to perform the actions of the methods and systems described herein.

Implementations of this aspect of the disclosure may include one or more of the following features. The implantable neurostimulator where the control circuit is in electrical communication with a therapy delivery circuit and causes the therapy delivery circuit to deliver electrical energy to the bi-polar electrodes upon a determination that the EMG signal is below a threshold value. The implantable neurostimulator where the control circuit is in electrical communication with a therapy delivery circuit and causes the therapy delivery circuit to deliver electrical energy to the bi-polar electrodes upon a determination that the EMG signal is below a threshold value and a heart rate detected by the sensor is below a threshold. The implantable neurostimulator where the control circuit is in electrical communication with a therapy delivery circuit and causes the therapy delivery circuit to deliver electrical energy to the bi-polar electrodes upon a determination that the EMG signal is below a threshold value and a motion sensor determines that the INS is not moving. The implantable neurostimulator where the control circuit is in electrical communication with a therapy delivery circuit and causes the therapy delivery circuit to deliver electrical energy to the bi-polar electrodes upon a determination that the EMG signal is below a threshold value and an acoustic sensor detects sounds consistent with snoring. The implantable neurostimulator where the control circuit is in electrical communication with a therapy delivery circuit and causes the therapy delivery circuit to deliver electrical energy to the bi-polar electrodes upon a determination that the EMG signal is below a threshold value and a temperature sensor detects a body temperature consistent with sleeping. The implantable neurostimulator where the control circuit is in electrical communication with a therapy delivery circuit and causes the therapy delivery circuit to deliver electrical energy to the bi-polar electrodes upon a determination that the EMG signal is below a threshold value and a breathing rate sensor detects a breathing rate consistent with sleeping.

A further aspect of the disclosure is directed to a system including: an implantable neurostimulator (INS), including a lead having at least one pair of bi-polar electrodes, and a pulse generator in electrical communication with the bi-polar electrodes, the pulse generator including a sensor, a memory, a control circuit, and a telemetry circuit; an external programmer in communication with the INS via the telemetry circuit; a server in communication with the external programmer and including thereon an application configured to receive sensor data from the INS from the external programmer and assess a quality of the sleep of a patient in which the INS is implanted based on the received sensor data; and a remote computer in communication with the server and configured to present an assessment of the quality of sleep of the patient. Other embodiments of this aspect include corresponding computer systems, apparatus, and computer programs recorded on one or more computer storage devices, each configured to perform the actions of the methods and systems described herein.

Implementations of this aspect of the disclosure may include one or more of the following features. The system further including a user interface presented on the external programmer and configured to receive a variety of self-reported data entered by the patient. The system where the application is further configured to assess the quality of the sleep of a patient in which the INS is implanted based on the received sensor data and the self-reported data. The system where the assessment of the quality of sleep is presented in the form of a sleep score. The system where the application is configured to assess the quality of the sleep of a patient in which the INS is implanted based on the received sensor data and self-reported data entered via a user interface on the external programmer and to determine a set of suggested updated stimulation parameters for the INS. The system where the received sensor data includes one or more of a tonal state of protrusor muscles, heartrate, blood pressure, blood oxygen saturation, patient temperature, arousals, awakenings, and electromyography data. The system where the updated stimulation parameters are available of review, acceptance, modification, or rejection on the remote computer. The system where upon acceptance or modification of the updated stimulation parameters, the updated stimulation parameters are transmitted to the external programmer. The system where the external programmer transmits the updated stimulation parameters to the INS.

Still a further aspect of the disclosure is directed to a non-claimed method of providing feedback for an implantable neurostimulator (INS), including receiving sensor data from an INS having at least one lead implanted in a protrusor muscle of a patient. The method also includes receiving self-reporting data entered via a user interface. The method also includes analyzing the sensor and self-reported data to determine a sleep score. The method also includes recording the sleep score. The method also includes presenting the sleep score for analysis. Other embodiments of this aspect include corresponding computer systems, apparatus, and computer programs recorded on one or more computer storage devices, each configured to perform the actions of the methods and systems described herein.

Implementations of this aspect of the disclosure may include one or more of the following features. The method further including providing suggestions for updating stimulation parameters of the INS. The method further including transmitting updated stimulation parameters to an external programmer associated with the INS and updating the INS. The method further including analyzing with an artificial intelligence the self-reported data and sensor to determine whether a reversion to the stimulation parameters of the INS prior to the update is necessary. Implementations of the described techniques may include hardware, a method or process, or computer software on a computer-accessible medium, including software, firmware, hardware, or a combination of them installed on the system that in operation causes or cause the system to perform the actions. One or more computer programs can be configured to perform particular operations or actions by virtue of including Instructions that, when executed by data processing apparatus, cause the apparatus to perform the actions.

### BRIEF DESCRIPTION OF THE DRAWINGS

FIG. 1 is a conceptual diagram of an implantable neurostimulator (INS) for delivering OSA therapy;
FIG. 2 is a conceptual diagram of a pulse generator included in INS of FIG. 1;
FIG. 3 is a diagram of the distal portion of the lead of the INS of FIG. 1 deployed for delivering OSA therapy according to one aspect of the disclosure;
FIG. 4 is a diagram of the distal portion of a two lead INS deployed for delivering OSA therapy according to a further aspect of the disclosure;
FIG. 5 is a timing diagram illustrating a method performed by the system of FIG. 1 for delivering selective stimulation to the protrusor muscles for promoting upper airway patency during sleep according to one example; and
FIG. 6 is a timing diagram of a method for delivering OSA therapy by the system of FIG. 1 according to another example.
FIG. 7A depicts an electromyography plot of the tongue compared to pharyngeal pressure during breathing;
FIG. 7B depicts the change in electromyography activity from an awake to sleep state for both normal subjects and those suffering from OSA;
FIG. 8 depicts electromyography of the tongue at different sleep and wakefulness states;
FIG. 9 depicts steps of signal processing required to transform raw electromyography signals into useable data streams in accordance with the present disclosure;
FIG. 10 depicts a simplified system for collecting, transmitting, and analyzing data derived from or directed to and INS;
FIG. 11 depicts a flow chart for collecting, transmitting, and analyzing data derived from or directed to and INS.

### DETAILED DESCRIPTION

An implantable neurostimulator (INS) system for delivering electrical stimulation to the lingual muscles of the tongue, in particular the protrusor muscles, for the treatment of OSA is described herein. Electrical stimulation is delivered to cause the tongue of a patient to be in a protruded state, during sleep, to avoid or reduce upper airway obstruction. As used herein, the term, "protruded state" with regard to the tongue refers to a position that is moved forward and/or downward compared to the non-stimulated position or a relaxed position. Those of skill in the art will recognize that to be in a protruded state does not require the tongue to be coming out of the mouth of the patient, indeed it is preferable that the tongue not extend out of the mouth of the patient, but only be advanced forward to a point where obstruction of the airway is mitigated or eliminated. The protruded state is a state associated with the recruitment of protrusor muscles of the tongue (also sometimes referred to as "protruder" muscles of the tongue) including the genioglossus and geniohyoid muscles. A protruded state may be the opposite of a retracted and/or elevated position associated with the recruitment of the retractor muscles, e.g., styloglossus and hyoglossus muscles, which retract and elevate the tongue. Electrical stimulation is delivered to cause the tongue to move to and maintain a protruded state to prevent collapse, open or widen the upper airway of a patient to promote unrestricted or at least reduced restriction of airflow during breathing.

Current INS systems must be turned on and off manually by the patient when they go to sleep and wake up. As will be appreciated, manual switching is not always a desirable feature in an implantable device associated with sleeping. In accordance with one aspect of the disclosure the INS only need to be turned on when there is a loss of lingual muscle tone (i.e., the protruder muscles are not being sufficiently stimulated naturally). The loss of lingual muscle tone increases the susceptibility of the patient to experience an OSA event. Accordingly, one aspect of the disclosure is directed to systems and methods for assessing the muscle tone of the protruder muscles, based on the tonal state determining that the patient is in need of therapy, and applying the needed therapy. The result is a therapy system which will be more "natural" and convenient for the patient and increase therapy compliance.

A further aspect of the disclosure is directed to systems and methods (non-claimed) of utilizing the sensed tonal state of the protrusor muscles along with a variety of self-reported and detected patient data to develop a patient feedback sleep score for consultation, stimulation modification, and health care reimbursement support.

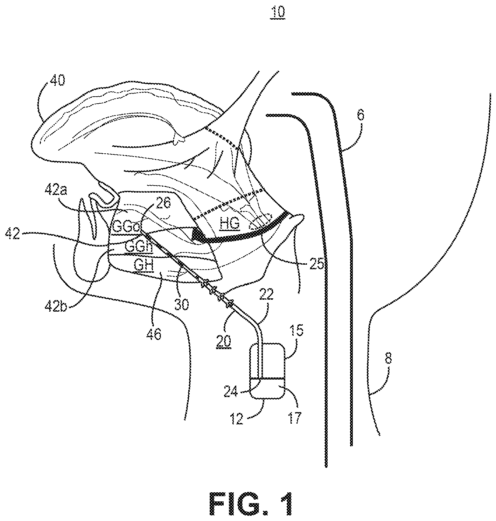

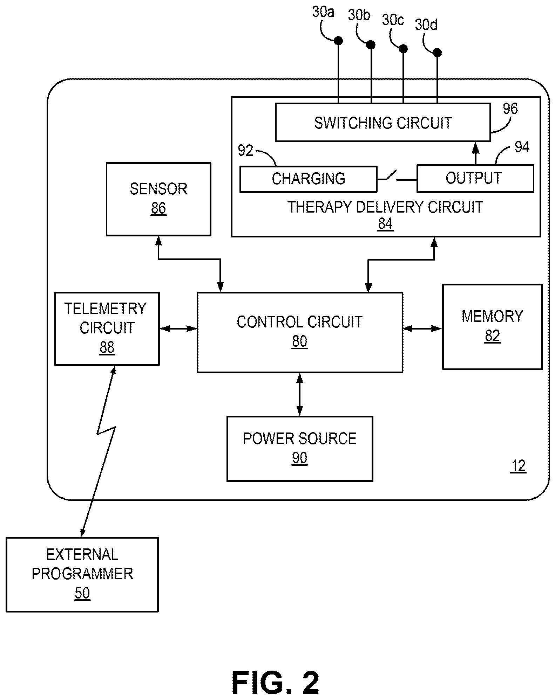

FIG. 1 is a conceptual diagram of an implantable neurostimulator (INS) system for delivering OSA therapy. The INS system 10 includes at least one electrical lead 20 and a pulse generator 12. Pulse generator 12 includes a housing 15 enclosing circuitry including a control circuit, therapy delivery circuit, optional sensor, a battery, and telemetry circuit as described below in conjunction with FIG. 2. A connector assembly 17 is hermetically sealed to housing 15 and includes one or more connector bores for receiving at least one medical electrical lead used for delivering OSA therapy and, in some examples, for sensing physiological conditions such as electromyogram (EMG) signals and the like. As depicted in FIG. 1 the pulse generator 12 is implanted in the neck of the patient 8. The instant disclosure is not so limited, and the pulse generator 12 may be located in other locations such as in the chest area or other areas known to those of skill in the art.

Lead 20 includes a flexible, elongate lead body 22 that extends from a lead proximal end 24 to a lead distal end 26. At least two electrodes 30 are carried along a lead distal portion adjacent lead distal end 26 that are configured for insertion within the protrusor muscles 42a, 42b and 46 of the patient's tongue 40. The electrodes 30 are configured for implantation within soft tissue such as musculature proximate to the medial branches of one or both hypoglossal nerves (HGN) that innervate the protrusor muscles of the tongue. The electrodes may be placed approximately 5mm (e.g., from 2mm to 8 mm) from a major trunk of the HGN. As such, the electrodes 30 may be referred to herein as "intramuscular electrodes," in contrast to an electrode that is placed on or along a nerve trunk or branch, such as a cuff electrode, used to directly stimulate the nerve trunk or branch. Lead 20 may be referred to herein as an "intramuscular lead" since the lead distal end and electrodes 30 are configured for advancement through the soft tissue, which may include the protrusor muscle tissue, to anchor electrodes 30 in proximity of the HGN branches that innervate the protrusor muscles 42a, 42b and 46. The term "intramuscular" with regard to electrodes 30 and lead 20 is not intended to be limiting, however, since the electrodes 30 may be implanted in connective tissue or other soft tissue proximate the medial HGN and its branches. One or more electrodes 30 may be placed in an area of protrusor muscles 42a, 42b and 46 that include motor points, where each nerve axon terminates in the muscle (also called the neuromuscular junction). The motor points are not at one location but spread out in the protrusor muscles. Leads 20 may be implanted such that one or more electrodes 30 may be generally in the area of the motor points (e.g., such that the motor points are within 1 to 10 mm from one or more electrodes 30).

The protrusor muscles are activated by electrical stimulation pulses generated by pulse generator 12 and delivered via the intramuscular electrodes 30 to move tongue 40 forward, to promote a reduction in obstruction or narrowing of the upper airway 6 during sleep. As used herein, the term "activated" with regard to the electrical stimulation of the protrusor muscles refers to electrical stimulation that causes depolarization or an action potential of the cells of the nerve (e.g., hypoglossal nerve(s)) innervating the protrusor muscles and motor points and subsequent depolarization and mechanical contraction of the protrusor muscle cells. In some cases, the muscles may be activated directly by the electrical stimulation pulses. The protrusor muscles that may be activated by stimulation via intramuscular electrodes 30 may include at least one or both of the right and/or left genioglossus muscle (GG) 42, which includes the oblique compartment (GGo) 42a and the horizontal compartment (GGh) 42b (referred to collectively as GG 42) and/or the right and/or left geniohyoid muscle (GH) 46. The GG muscle and GH muscle are innervated by a medial branch of the HGN (also referred to as the XIIth cranial nerve), while the hyoglossus and styloglossus muscles, which cause retraction and elevation of the tongue, are innervated by a lateral branch of the HGN.

The multiple distal electrodes 30 may be used to deliver bilateral or unilateral stimulation to the GG 42 and/or the GH 46 muscles via the medial branch of the HGN or branches thereof, also referred to herein as the "medial HGN." Distal electrodes 30 may be switchably coupled to output circuitry of pulse generator 12 to enable delivery of electrical stimulation pulses in a manner that selectively activates the right and left protrusor muscles in a cyclical or alternating pattern to avoid muscle fatigue while maintaining upper airway patency. Additionally or alternatively, electrical stimulation may be delivered to selectively activate the GG 42 and/or GH 46 muscles or portions thereof during unilateral stimulation of the left or right protrusor muscles.

The lead proximal end 24 includes a connector (not shown in FIG. 1) that is coupleable to connector assembly 17 of pulse generator 12 to provide electrical connection between circuitry enclosed by the housing 15 of pulse generator 12, e.g., including therapy delivery circuitry and control circuitry as described below in conjunction with FIG. 2. The lead body 22 encloses electrical conductors extending from each of the distal electrodes 30 to the proximal connector at proximal end 24 to provide electrical connection between output circuitry of pulse generator 12 and the electrodes 30.

Though shown in Fig. 1 as separate from and extending from the pulse generator 12, the lead 20 could be integrated into a portion of the pulse generator 12, and merely be an exposed surface of the pulse generator 12. In such an embodiment, the pulse generator would be implanted proximate the lingual muscles under the chin of the patient. In contrast the embodiment shown in FIG. 1 allows for more flexibility in the placement of the pulse generator in the neck or pectoral region of the patient.

FIG. 2 is a schematic diagram of pulse generator 12. Pulse generator 12 includes a control circuit 80, memory 82, therapy delivery circuit 84, a sensor 86, telemetry circuit 88 and power source 90. Power source 90 may include one or more rechargeable or non-rechargeable batteries for supplying electrical current to each of the control circuit 80, memory 82, therapy delivery circuit 84, sensor 86 and telemetry circuit 88. While power source 90 is shown in communication only with control circuit 80 for the sake of clarity, it is to be understood that power source 90 provides power as needed to each of the circuits and components of pulse generator 12 as needed. For example, power source 90 provides power to therapy delivery circuit 84 for generating electrical stimulation pulses.

Sensor 86 may include one or more separate sensors for monitoring a patient condition. These sensors may include one or more accelerometers, inertial measurement units (IMU), fiber-Bragg gratings (e.g., shape sensors), optical sensors, acoustic sensors, pulse oximeters, and others without departing from the scope of the disclosure and as will be described in greater detail below. In one aspect of the disclosure sensor 86 is configured as, among other things, a patient posture sensor. Patient posture data may be stored in memory 82 from the detected posture states of patient when sensor 86 is included, and may be presented on a display of external programmer 50, e.g., as generally described in U.S. Pat. No. 9,662,045 (Skelton, et al.).

Additionally or alternatively, the sensor 86 may detect a signal that is correlated to the movement of the patient's tongue into and out of a protruded state. This signal may be used to detect adequate protrusion and/or fatigue of the stimulated muscle for use in controlling the duty cycle, pulse amplitude and/or stimulating electrode vector of the electrical stimulation therapy delivered by therapy delivery circuit 84.

The functional blocks shown in FIG. 2 represent functionality included in a pulse generator 12 configured to delivery an OSA therapy and may include any discrete and/or integrated electronic circuit components that implement analog and/or digital circuits capable of producing the functions attributed to a pulse generator herein. The various components may include an application specific integrated circuit (ASIC), an electronic circuit, a processor (shared, dedicated, or group) and memory that execute one or more software or firmware programs, a combinational logic circuit, state machine, or other suitable components or combinations of components that provide the described functionality. Providing software, hardware, and/or firmware to accomplish the described functionality in the context of any modern medical device system, given the disclosure herein, is within the abilities of one of skill in the art.

Control circuit 80 communicates, e.g., via a data bus, with memory 82, therapy delivery circuit 84, telemetry circuit 88 and sensor 86 (when included) to control OSA therapy delivery and other pulse generator functions. As disclosed herein, control circuit 80 may pass control signals to therapy delivery circuit 84 to cause therapy delivery circuit 84 to deliver electrical stimulation pulses via electrodes 30 according to a therapy protocol that may include selective stimulation patterns of right and left portions of the GG and GH muscles and/or proximal and distal portions of the GG and GH muscles. Control circuit 80 may further be configured to pass therapy control signals to therapy delivery circuit 84 including stimulation pulse amplitude, stimulation pulse width, stimulation pulse number and frequency of a stimulation pulse train.

Memory 82 may store instructions for execution by a processor included in control circuit 80, stimulation control parameters, and other device-related or patient-related data. Control circuit 80 may retrieve therapy delivery control parameters and a therapy delivery protocol from memory 82 to enable control circuit 80 to pass control signals to therapy delivery circuit 84 for controlling the OSA therapy. Memory 82 may store historical data relating to therapy delivery for retrieval by a user via telemetry circuit 88. Therapy delivery data or information stored in memory 82 may include therapy control parameters used to deliver stimulation pulses as well as delivered therapy protocol(s), hours of therapy delivery or the like. Patient related data, such as that received from the sensor 86 signal may be stored in memory 82 for retrieval by a user.

Therapy delivery circuit 84 may include a charging circuit 92, an output circuit 94, and a switching circuit 96. Charging circuit 92 may include one or more holding capacitors that are charged using a multiple of the battery voltage of power source 90, for example. The holding capacitors are switchably connected to output circuit 94, which may include one or more output capacitors that are coupled to a selected bipolar electrode pair via switching circuit 96. The holding capacitor(s) are charged to a programmed pacing pulse voltage amplitude by charging circuit 92 and discharged across the output capacitor for a programmed pulse width. Charging circuit 92 may include capacitor charge pumps or an amplifier for the charge source to enable rapid recharging of holding capacitors included in charging circuit 92. Therapy delivery circuit 84 responds to control signals from control circuit 80 for generating and delivering trains of pulses to produce sustained tetanic contraction of the GG and/or GH muscles or portions thereof to move the tongue forward and avoid upper airway obstruction.

Output circuit 94 may be selectively coupled to bipolar pairs of electrodes 30a-30d via switching circuit 96. Switching circuit 96 may include one or more switches activated by timing signals received from control circuit 80. Electrodes 30a-30d may be selectively coupled to output circuit 94 in a time-varying manner to deliver stimulation to different portions of the protrusor muscles at different time to avoid fatigue, without requiring stimulation to be withheld completely. Switching circuit 96 may include a switch array, switch matrix, multiplexer, or any other type of switching device(s) suitable to selectively couple therapy delivery circuit 84 to bipolar electrode pairs selected from electrodes 30. Bipolar electrode pairs may be selected one at a time or may be selected two or more at time to allow overlapping stimulation of two or more different portions of the protrusor muscles. Overlapping stimulation times of two portions of the protrusor muscles, for example left and right or proximal and distal may maintain a forward position of the tongue and allow a ramping up and ramping down of the electrical stimulation being delivered to two different portions of the protrusor muscles.

Telemetry circuit 88 is optional but may be included to enable bidirectional communication with an external programmer 50. A user, such as the patient 8, may manually adjust therapy control parameter settings, e.g., as described in Medtronic's Patient Programmer Model 37642. The patient may make limited programming changes such as small changes in stimulation pulse amplitude and pulse width. The patient may turn the therapy on and off or set timers to turn the therapy on or off using external programmer 50 in wireless telemetric communication with telemetry circuit 88.

In other examples, a user, such as a clinician, may interact with a user interface of an external programmer 50 to program pulse generator 12 according to a desired OSA therapy protocol. For example, a Physician Programmer Model 8840 available from Medtronic, Inc., Minneapolis, MN, may be used by the physician to program pulse generator 12 for delivering electrical stimulation.

Programming of pulse generator 12 may refer generally to the generation and transfer of commands, programs, or other information to control the operation of pulse generator 12. For example, external programmer 50 may transmit programs, parameter adjustments, program selections, group selections, or other information to control the operation of pulse generator 12, e.g., by wireless telemetry. As one example, external programmer 50 may transmit parameter adjustments to support therapy changes. As another example, a user may select programs or program groups. A program may be characterized by an electrode combination, electrode polarities, voltage or current amplitude, pulse width, pulse rate, therapy duration, and/or pattern of electrode selection for delivering patterns of alternating portions of the protrusor muscles that are being stimulated. A group may be characterized by multiple programs that are delivered simultaneously or on an interleaved or rotating basis. These programs may adjust output parameters or turn the therapy on or off at different time intervals.

External programmer 50 may present patient related and/or device related data retrieved from memory 82 via telemetry circuit 88. Additionally or alternatively, external programmer 50 may present sleep sound or motion data stored in memory 82 as determined from signals from sensor 86. Further, the time periods in which the patient is lying down can be acquired based on patient posture detection using sensor 86 and a history of such data can be stored into memory 82 and retrieved and displayed by external programmer 50.

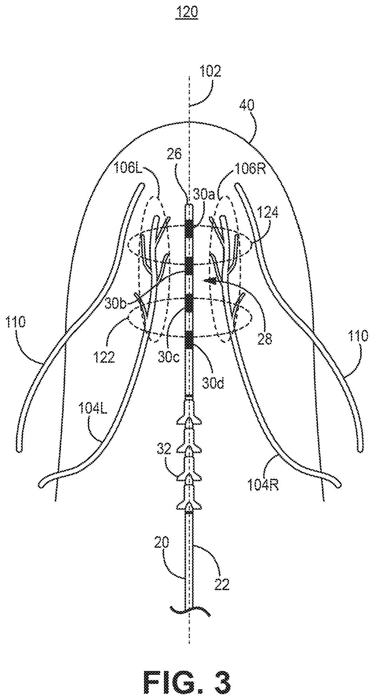

FIG. 3 depicts a single intramuscular lead 20 inserted into the tongue 40 of a patient. Lead 20 may include two or more electrodes, and in the example shown lead 20 includes four electrodes 30a, 30b, 30c, and 30d (collectively referred to as "electrodes 30") spaced apart longitudinally along lead body 22. Lead body 22 is a flexible lead body which may define one or more lumens within which insulated electrical conductors extend to a respective electrode 30a-30d. The distal most electrode 30a may be adjacent or proximate to lead distal end 26. Each of electrodes 30b, 30c and 30d are spaced proximally from the respective adjacent electrode 30a, 30b and 30c by a respective interelectrode distance 34, 35 and 36.

Each electrode 30a-30d is shown have equivalent electrode lengths 31. In other examples, however, electrodes 30a-30d may have electrode lengths 31 that are different from each other in order to optimize placement of the electrodes 30 or the resulting electrical field of stimulation relative to targeted stimulation sites corresponding to left and right portions of the HGN or branches thereof and/or motor points of the GG and GH muscles. The interelectrode spacings between electrodes 30a, 30b, 30c, and 30d are shown to be approximately equal in FIG. 3, however they may also be different from each other in order to optimize placement of electrodes 30 relative to the targeted stimulation sites or the resulting electrical field of stimulation relative to targeted stimulation sites corresponding to left and right hypoglossal nerves or branches of hypoglossal nerves and/or motor points of protrusor muscles 42a, 42, or 46.

In some examples, electrodes 30a and 30b form an anode and cathode pair for delivering bipolar stimulation in one portion of the protrusor muscles, e.g., either the left or right GG and/or GH muscles or either a proximal or distal portion of the GG and/or GH muscles. Electrodes 30c and 30d may form a second anode and cathode pair for delivering bipolar stimulation in a different portion of the protrusor muscles (e.g., the other of the left or right portions or the other of the proximal or distal portions). Accordingly, the interelectrode spacing 35 between the two bipolar pairs 30a-30b and 30c-30d may be different than the interelectrode spacing 34 and 36 between the anode and cathode within each bipolar pair 30a-30b and 30c-30d.

In one example, the total distance encompassed by electrodes 30a-30d along the lead body 22 may be about 20 millimeter, 25 millimeters, or 30 millimeters as examples. In one example, the total distance is between 20 and 22 millimeters. The interelectrode spacings between a proximal electrode pair 30c-30d and a distal electrode pair 30a-30b, respectively, may be between 2 and 6 mm, including all integer values therebetween. The interelectrode spacing separating the distal and proximal pairs 30a-30b and 30c-30d may be the same or different from each other and the spacing between individual electrodes of any such pair.

In the example shown, each of electrodes 30a-30d is shown as a circumferential ring electrode which may be uniform in diameter with lead body 22. In other examples, electrodes 30 may include other types of electrodes such as a tip electrode, a helical electrode, a coil electrode, a segmented electrode, a button electrode as examples. For instance, the distal most electrode 30a may be provided as a tip electrode at the lead distal end 26 with the remaining three electrodes 30b, 30c and 30d being ring electrodes. When electrode 30a is positioned at the distal end 26, electrode 30a may be a helical electrode configured to screw into the muscle tissue at the implant site to additionally serve as a fixation member for anchoring the lead 20 at the targeted therapy delivery site. In other examples, one or more of electrodes 30a-d may be a hook electrode or barbed electrode to provide active fixation of the lead 20 at the therapy delivery site.

Lead 20 may include one or more fixation member 32 for minimizing the likelihood of lead migration. In the example shown, fixation member 32 includes multiple sets of tines which engage the surrounding tissue when lead 20 is positioned at the target therapy delivery site. The tines of fixation member 32 may extend radially and proximally at an angle relative to the longitudinal axis of lead body 22 to prevent or reduce retraction of lead body 22 in the proximal direction. Tines of fixation member 32 may be collapsible against lead body 22 when lead 20 is held within the confines of a lead delivery tool, e.g., a needle or introducer, used to deploy lead 20 at the target implant site. Upon removal of the lead delivery tool, the tines of fixation member 32 may spread to a normally extended position to engage with surrounding tissue and resist proximal and lateral migration of lead body 22. In other examples, fixation member 32 may include one or more hooks, barbs, helices, or other fixation mechanisms extending from one or more longitudinal locations along lead body 22 and/or lead distal end 26. Fixation member 32 may partially or wholly engage the GG, GH muscles and/or other muscles below the tongue, and/or other soft tissues of the neck, e.g., fat and connective tissue, when proximal end of lead body 20 is tunneled to an implant pocket of pulse generator 12. In other examples, fixation member 32 may include one or more fixation mechanisms located at other locations than the location shown in FIG. 3, including at or proximate to distal end 26, between electrodes 30, or otherwise more distally or more proximally than the location shown. The implant pocket of pulse generator 12 may be along the patient's neck 8 (see FIG. 1) in the chest, or in another location as deemed appropriate by the surgeon performing the implantation. Accordingly the length of the elongated lead body 22 from distal end 26 to the lead proximal end 24 (FIG. 1) may be selected to extend from the target therapy delivery site in the protrusor muscles to a location along the patient's neck where the pulse generator 12 is implanted. This length may be up to 10 cm or up to 20 cm as examples but may generally be 25 cm or less, though longer or shorter lead body lengths may be used depending on the anatomy and size of the individual patient.

FIG. 2 is a conceptual diagram 120 of the lead 20 deployed for delivering OSA therapy according to another example. In this example, lead 20 carrying electrodes 30 is advanced approximately along or parallel to midline 102 of tongue 40. In the example shown, lead body 22 is shown approximately centered along midline 102, however in other examples lead body 22 may be laterally offset from midline 102 in the left or right directions but is generally medial to both of the left HGN 104L and the right HGN 104R. The distal end 26 of lead 20 may be inserted inferiorly to the body of tongue 40, e.g., at a percutaneous insertion point along the submandibular triangle, in the musculature below the floor of the oral cavity. The distal end 26 is advanced to position electrodes 30 medially to the left and right HGNs 104L and 104R, e.g., approximately midway between the hyoid bone the mental protuberance (chin). An electrical field produced by stimulation pulses delivered between any bipolar pair of electrodes selected from electrodes 30 may encompass a portion of both the left target region 106L and the right target region 106R to produce bilateral stimulation of the HGNs 104L and 104R and therefore bilateral recruitment of the protrusor muscles. Bilateral recruitment of the protrusor muscles may provide greater airway opening than unilateral stimulation that is generally performed using a nerve cuff electrode along the HGN. For example, electrical stimulation pulses delivered using electrodes 30a and 30b may produce electrical field 122 (shown conceptually) encompassing a portion of both of the left and right target regions 106L and 106R. Electrical stimulation pulses delivered using electrodes 30c and 30d may produce electrical field 124 (shown conceptually) encompassing a portion of both of the left and right target regions 106L and 106R. The portions of the left and right target regions 106L and 106R encompassed by electrical field 122 are posterior portions relative the portions of the left and right target regions 106L and 106R encompassed by electrical field 124.

In some examples, electrical stimulation is delivered by pulse generator 12 by sequentially selecting different electrode pairs from among the available electrodes 30 to sequentially recruit different bilateral anterior and bilateral posterior portions of the HGNs 104L and 104R. This electrode selection may result in recruitment of different anterior and posterior portions of the protrusor muscles. The sequential selection of different electrode pairs may be overlapping or non-overlapping. The electrical stimulation is delivered throughout an extended time period encompassing multiple respiratory cycles independent of the timing of respiratory cycles to maintain a protruded state of tongue 40 from the beginning of the time period to the end of the time period. The electrodes 30 may be selected in bipolar pairs comprising the most distal pair 30a and 30b, the outermost pair 30a and 30d, the innermost pair 30b and 30c, the most proximal pair 30c and 30d or alternating electrodes along lead body 22, e.g., 30a and 30c or 30b and 30d. Sequential selection of two or more different electrode pairs allows for sequential recruitment of different portions of the protrusor muscles to reduce the likelihood of fatigue.

In some examples, electrical stimulation delivered using an electrode pair, e.g., 30a and 30b, that is relatively more distal along distal lead portion 28 and implanted relatively anteriorly along tongue 40 may recruit a greater portion of anterior muscle fibers, e.g., within the GG muscle. Electrical stimulation delivered using an electrode pair, e.g., 30c and 30d, that is relatively more proximal along distal lead portion 28 and implanted relatively posteriorly along tongue 40 may recruit a greater portion of posterior muscle fibers, e.g., within the GH muscle. Sequential selection of electrodes 30 for delivering electrical stimulation pulses allows sequential recruitment in overlapping or non-overlapping patterns of anterior and posterior portions of the protrusor muscles to sustain the tongue in a protruded state throughout the extended time period while reducing or avoiding muscle fatigue.

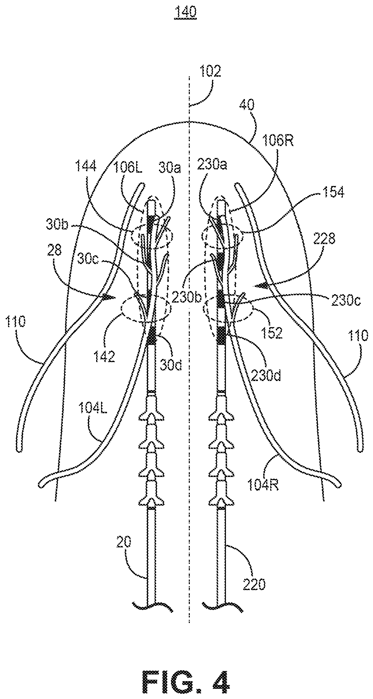

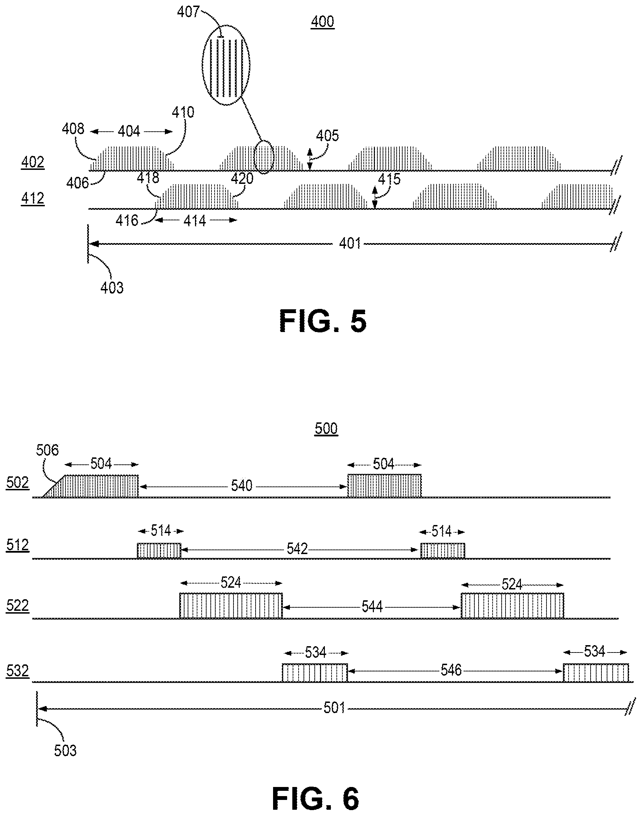

FIG. 4 is a conceptual diagram of the distal portion of a dual lead system for delivering OSA therapy. In this example, one lead 20 is advanced anteriorly approximately parallel to midline 102 and offset, e.g. by 5-8 millimeters to the left of midline 102, to position distal portion 28 and electrodes 30 in or adjacent to the left target region 106L. A second lead 220 is advanced anteriorly approximately parallel to midline 102 but offset laterally to the right of midline 102 to position distal portion 228 and electrodes 230 in or adjacent the right target region 106R. Lead 20 may be inserted from a left lateral or posterior approach of the body of tongue 40, and lead 230 may be inserted from a right lateral or posterior approach of the body of tongue 40. In other examples, both leads 20 and 220 may be inserted from only a left or only a right approach with one lead traversing midline 102 to position the electrodes 30 or 230 along the opposite side of midline 102 from the approaching side. Lead 20 and/or lead 220 may be advanced at an oblique angle relative to midline 102 but may not cross midline 102. In other examples, one or both leads 20 and 220 may approach and cross midline 102 at an oblique angle such that one or both of distal portions 28 and 228 extend in or adjacent to both the right and left target regions 106L and 106R, similar to the orientation shown in FIG. 6.

In the example shown, relatively more localized control of the recruitment of left, right, anterior and posterior portions of the protrusor muscles may be achieve by selecting different electrode pairs from among the electrodes 30a through 30d and 230a through 230d. For example, any combination of electrodes 30a through 30d may be selected for delivering electrical stimulation pulses to the left portions of the protrusor muscles. More distal electrodes 30a and 30b may be selected for stimulation of more anterior portions of the left protrusor muscles (corresponding to electrical field 144) and more proximal electrodes 30c and 30d may be selected for stimulation of more posterior portions of the left protrusor muscles (corresponding to electrical field 142). Any combination of electrodes 230a through 230d may be selected for delivering electrical stimulation pulses to the right portions of the protrusor muscles. More distal electrodes 230a and 230b may be selected for stimulation of more anterior portions of the right protrusor muscles (corresponding to electrical field 154) and more proximal electrodes 230c and 230d may be selected for stimulation of more posterior portions of the right protrusor muscles (corresponding to electrical field 152).

Switching circuit 96 may be configured to select electrode pairs that include one electrode on one of leads 20 or 220 and another electrode on the other lead 20 or 220 to produce an electrical field (not shown) that encompasses portions of both the left target region 106L and the right target region 106R simultaneously for bilateral stimulation. Any combination of the available electrodes 30a through 30d and electrodes 230a through 230d may be selected as two or more bipolar pairs, which are selected in a repeated, sequential pattern to sequentially recruit different portions of the two target regions 106L and 106R. The sequential selection of electrode pairs may be overlapping or non-overlapping, but electrical stimulation pulses are delivered without interruption at one or more selected frequencies throughout an extended time period to maintain tongue 40 in a protruded state from the beginning of the time period to the end of the time period, encompassing multiple respiratory cycles.

In the example of FIG. 4 including two leads, two pairs of electrodes may be selected simultaneously and sequentially with one or more other pairs of electrodes. For example, electrodes 30a and 30b may be selected as one bipolar pair and electrodes 230c and 230d may be selected as a second bipolar pair for simultaneous stimulation of the left, anterior portion of the target region 106L and the right posterior portion of the target region 106R. The electrodes 30c and 30d may be selected as the next bipolar pair from lead 20, simultaneously with electrodes 230a and 230b selected as the next bipolar pair from lead 220. In this way, electrical stimulation may be delivered bilaterally, alternating between posterior and anterior regions on each side. The anterior left (30a and 30b) and posterior right (230c and 230d) bipolar pairs may be selected first, and the posterior left (30c and 30d) and anterior right (230a and 230b) bipolar pairs may be selected second in a repeated, alternating fashion to maintain tongue 40 in a protruded state continuously during an extended time period encompassing multiple respiratory cycles. In other examples, both of the anterior pairs (30a-30b and 230a-230b) may be selected simultaneously first, and both the posterior pairs (30c-30d and 230c-230d) may be selected simultaneously second, sequentially following the anterior pairs. In this way, continuous bilateral stimulation may be achieved while sequentially alternating between posterior and anterior portions to avoid or reduce fatigue. In contrast to other OSA therapy systems that rely on a sensor for sensing the inspiratory phase of respiration to coordinate the therapy with the inspiratory phase, the intramuscular electrodes 30 positioned to stimulate different portions of the protrusor muscles do not require synchronization to the respiratory cycle. Alternation of stimulation locations within the protrusor muscles allows different portions of the muscles to rest while other portions are activated to avoid collapse of the tongue against the upper airway while also avoiding muscle fatigue.

It is to be understood that more or fewer than the four electrodes shown in the examples presented herein may be included along the distal portion of a lead used in conjunction with the OSA therapy techniques disclosed herein. A lead carrying multiple electrodes for delivering OSA therapy may include 2, 3, 5, 6 or other selected number of electrodes. When the lead includes only two electrodes, a second lead having at least one electrode may be included to provide at least two different bipolar electrode pairs for sequential stimulation of different portions of the right and/or left medial HGNs. Furthermore, while the selected electrode pairs are generally referred to herein as "bipolar pair" including one cathode and one return anode, it is recognized that three or more electrodes may be selected at a time to provide desired electrical field or stimulation vector for recruiting a desired portion of the protrusor muscles. Accordingly, the cathode of a bipolar "pair" may include one or more electrodes selected simultaneously from the available electrodes and/or the anode of the bipolar "pair" may include one or more electrodes selected simultaneously from the available electrodes.

FIG. 5 timing diagram illustrating a method performed by pulse generator 12 for delivering selective stimulation to the protrusor muscles for promoting upper airway patency during sleep according to one example. Electrical stimulation is delivered over a therapy time period 401 having a starting time 403 and an ending time (not shown). Electrical stimulation pulses that are delivered when pulse generator sequentially selects a first bipolar electrode pair 402 and a second bipolar electrode pair 412 in an alternating, repeating manner are shown. The first and second bipolar electrode pairs 402 and 412 may correspond to any two different electrode pairs described in the examples above in conjunction with FIGs. 3-4.

A first train of electrical pulses 406 is shown starting at the onset 403 or therapy time period 401. The first train of electrical pulses 406 is delivered using bipolar electrode pair 402 for a duty cycle time interval 404. The first train of electrical pulses 406 has a pulse amplitude 405 and pulse frequency, e.g., 20 to 50 Hz, defined by the interpulse intervals 407. The first train of electrical pulses 406, also referred to as "pulse train" 406, may have a ramp on portion 408 during which the pulse amplitude is gradually increased from a starting voltage amplitude up to pulse voltage amplitude 405. In other examples, the pulse width may be gradually increased. In this way the delivered pulse energy is gradually increased to promote a gentle transition from the relaxed, non-stimulated state to the protruded state of the tongue.

The train of electrical pulses 406 may include a ramp off portion 410 during which the pulse amplitude (and/or pulse width) is decremented from the pulse voltage amplitude 405 to an ending amplitude at the expiration of the duty cycle time interval 404. In other examples, pulse train 406 may include a ramp on portion 408 and no ramp off portion 410. In this case, the last pulse of pulse train 406 delivered at the expiration of duty cycle time interval 404 may be delivered at the full pulse voltage amplitude 405. Upon expiration of the duty cycle time interval 404, electrical stimulation delivery via bipolar electrode pair 402 is terminated.

In the example shown, a second electrode pair 412 is selected when duty cycle time interval 404 is expiring. The second electrode pair 412 may be selected such that delivery of electrical stimulation pulse train 416 starts a ramp on portion 418 that is simultaneous with the ramp off portion 410 of train 406. In other examples, the ramp on portion 418 of pulse train 416 may start at the expiration of the first duty cycle time interval 404. When pulse train 406 does not include a ramp off portion 410, the pulse train 416 may be started such that the ramp on portion 418 ends just before, just after or coincidentally with the expiration of duty cycle time interval 404. The second pulse train 416 has a duration of duty cycle time interval 414 and may end with an optional ramp off portion 420, which may overlap with the ramp on portion of the next pulse train delivered using the first electrode pair 402.

In this example, pulse trains 406 and 416 are shown to be equivalent in amplitude 405 and 415, pulse width, pulse frequency (and inter pulse interval 407), and duty cycle time interval 404 and 414. It is contemplated, however, that each of the stimulation control parameters used to control delivery of the sequential pulse trains 406 and 416 may be separately controlled and set to different values as needed to achieve a desired sustained protrusion of tongue 40 while avoiding or minimizing fatigue.

The sequential pulse trains 406 and 416 are delivered using two different electrode pairs 402 and 412 such that different portions of the protrusor muscles are recruited by the pulse trains 406 and 416 allowing one portion to rest while the other is being stimulated. However, pulse trains 404 and 406 occur in a sequential overlapping or non-overlapping manner such that electrical pulses are delivered at one or more selected frequencies for the entire duration of the therapy time period 401 to sustain the tongue in a protruded state throughout time period 401. It is to be understood that the relative down and/or forward position of the protruded tongue may shift or change as different electrode pairs are selected but the tongue remains in a protruded state throughout therapy time period 401.

At times, the pulse trains 404 and 406 may be overlapping to simultaneously recruit the left and right GG and/or GH muscles to create a relatively greater force (compared to recruitment of a single side) to pull the tongue forward to open an obstructed upper airway. In some cases, the overlapping pulse trains 404 and 406 may cause temporary fatigue of the protrusor muscles along the left or right side but the temporary fatigue may improve the therapy effectiveness to ensure an open upper airway during an apneic episode. Recovery from fatigue will occur between duty cycles and at the end of an apneic episode. Duty cycle lengths may vary between patients depending on the fatigue properties of the individual patient. Control circuit 80 may control the duty cycle on time in a manner that minimizes or avoids fatigue in a closed loop system using a signal from sensor 86, e.g., a motion sensor signal and or electromyography (EMG) signal correlated protrusor muscle contraction force and subsequent fatigue.

FIG. 6 is a timing diagram 500 of a non-claimed method for delivering OSA therapy by pulse generator 12 according to another example. In this example, a therapy delivery time period 501 is started at 503 with a ramp on interval 506 delivered using a first bipolar electrode pair 502. The ramp on interval 506 is followed by a duty cycle time interval 504. Upon expiration of the duty cycle time interval 504, a second bipolar electrode pair 512 is selected for delivering electrical stimulation pulses for a second duty cycle time interval 514. A third duty cycle time interval 524 starts upon the expiration of the second duty cycle time interval 514, and stimulation pulses are delivered by selecting a third bipolar electrode pair 522 different than the first two pairs 502 and 512. A fourth bipolar pair 532 is selected upon expiration of the third duty cycle time interval 524 and used to deliver stimulation pulses over the fourth duty cycle time interval 534. Upon expiration of the fourth duty cycle time interval 534, the sequence is repeated beginning with duty cycle time interval 504 again.

In this example, four different bipolar pairs are selected in sequence. The four different bipolar electrode pairs may differ by at least one electrode and/or the polarity of another bipolar electrode pair. For example, when a single quadripolar lead 20 is used, the four bipolar pairs may include 30a-30b, 30b-30c, 30c-30d and 30a-30d. The portions of the protrusor muscles recruited by the four different pairs may not be mutually exclusive since the electrical fields of the four different pairs may stimulate some of the same nerve fibers. Four different portions of the protrusor muscles may be recruited, which may include overlapping portions. The relatively long recovery periods 540, 542, 544 and 546 between respective duty cycle time intervals allows each different portion of the protrusor muscles to recover before the next duty cycle. When recruited muscle portions overlap between selected electrode pairs, the bipolar electrode pairs may be selected in a sequence that avoids stimulating the overlapping recruited muscle portions consecutively. All recruited muscle portions are allowed to recover during at least a portion of each respective recovery period 540, 54, 544 and/or 546. For example, if the bipolar electrode pair 502 and the bipolar electrode pair 522 recruit overlapping portions of the protrusor muscles, the recruited portions may still recover during the second duty cycle time interval 514 and during the fourth duty cycle time interval 534.

The duration of each duty cycle time interval, 504, 514, 524 and 534, may be the same or different from each other, resulting in the same or different overall duty cycles. For example, when four bipolar electrode pairs are sequentially selected, stimulation delivery for each individual pair may be a 25% duty cycle. In other examples, a combination of different duty cycles, e.g., 30%, 10%, 40% and 20%, could be selected in order to promote sustained protrusion of the tongue with adequate airway opening while minimizing or avoiding fatigue. The selection of duty cycle may depend on the particular muscles or muscle portions being recruited and the associated response (position) of the tongue to the stimulation for a given electrode pair selection.

The stimulation control parameters used during each of the duty cycle time intervals 504, 514, 524, and 534 for delivering electrical pulses using each of the different bipolar electrode pairs 502, 512, 522 and 532 may be the same or different. As shown, a different pulse voltage amplitude and a different interpulse interval and resulting pulse train frequency may be used. The pulse amplitude, pulse width, pulse frequency, pulse shape or other pulse control parameters may be controlled according to settings selected for each bipolar electrode pair.

In the example shown, one ramp on portion 506 of the stimulation protocol is shown at the onset of the therapy delivery time period 501. Once the stimulation is ramped up to position the tongue in a protruded position, no other subsequent duty cycle time intervals 504 (other than the first one), 514, 524 and 534 may include or be proceeded by a ramp on portion. In other examples, a ramp on portion may precede each duty cycle time interval (or be included in the duty cycle time interval as shown in FIG. 5) and may overlap with the preceding duty cycle time interval. No ramp off portions are shown in the example of FIG. 6. In other examples, ramp off portions may follow or be included in each duty cycle time interval 504, 514, 524 and 534 and may overlap with the onset of the next duty cycle time interval as shown in FIG. 5. In some examples, only the last duty cycle time interval (not shown in FIG. 6) may include or be immediately followed by a ramp off portion to gently allow the tongue to return to a relaxed position at the end of the therapy delivery time period 501.

Following implantation as depicted in FIGS 3 and 4 and calibration by the surgeon or other caregiver, the INS 10 is ready for use. In accordance with one aspect of the disclosure, the INS system 10 is manually switched on by the patient as part of their routine prior to sleeping. This may be a function of the external programmer 50, or another similar device that can communicate with the pulse generator 12 via the telemetry circuit 88. A delay period may be programmed into the software or firmware employed by the control circuit 80. The delay period allows the patient a period to fall asleep before therapy is begun. The period may be established for the patient based on a variety of factors, including an average time to sleep observed during, for example, a sleep study and may be adjusted by the patient via the external programmer 50. Without the delay period, the patient would immediately begin to experience the effects of stimulating the protrusor muscles, which though not dangerous or painful, can be observed and may be considered annoying to experience while awake.

As will be appreciated, manual switching as described above, is not always a desirable feature in an implantable device associated with sleeping. In a further aspect of the disclosure, OSA therapy may be started and stopped at scheduled times of day. Control circuit 80 may include a clock for scheduling the time that OSA therapy is started and stopped by therapy delivery circuit 84. Many patients, however, are not as rigorous regarding their schedules as would be desired to make the scheduling most effective. Further, the patient may find themselves at a social gathering or other affair at a time where they are normally scheduled for sleeping. Additionally, or alternatively, the patient may find themselves taking an unscheduled nap in a motor vehicle, plane, or train, and not have an opportunity to initiate or schedule therapy. Since OSA is often co-morbid with heart related diseases any instances of experiencing OSA can have complicating factors affecting the patient's heart. Thus, sensing of sleeping conditions and initiation of therapy are desirable. One aspect of the disclosure is directed to a mechanism of initiating therapy based on a detected state of the tone of the protrusor muscles.

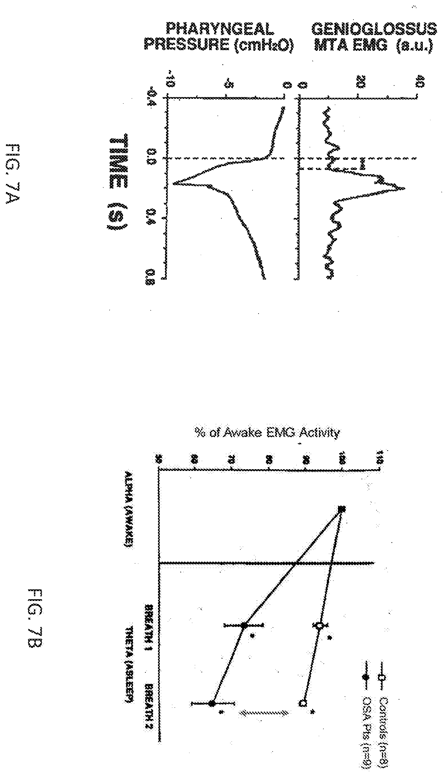

In accordance with the disclosure, and as noted above, the electrodes 30 either alone or in combination with the sensor 86 can be configured to detect electromyography (EMG) signals. Electromyography is a technique of evaluating and recording the electrical activity produced by skeletal muscles. An electromyograph detects the electrical potential generated by muscle cells when the cells are electrically or neurologically activated. FIG. 7A, in an upper plot depicts an EMG signal observed in a genioglossus muscle (GG) 42, in a patient during normal breathing. The lower plot in FIG. 7A depicts the pharyngeal pressure during the same period as the EMG signal in the upper plot. As can be seen in FIG. 7A, during breathing as the pharyngeal pressure drops, consistent with inhalation, the EMG signal significantly increases. Those of skill in the art will recognize that this increase in EMG signal during breathing, signifying stimulation of the muscles of the tongue such as the genioglossus muscle (GG) 42, ensures that the airway is not closed or collapsed easing the ability of the subject to take a breath. That is, at periods of high EMG signal the protrusor muscles have a contracted tonal state. At periods where there is a low EMG signal, the protrusor muscles have a relaxed tonal state.

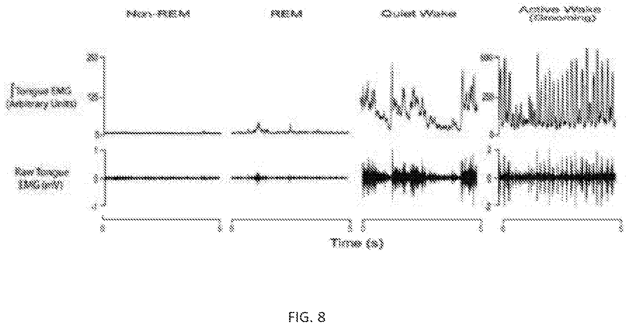

FIG. 7B depicts a comparison of observed EMG signals observed in the protrusor muscles of two sets of subjects. For all subjects, the EMG signal declines when the subject is in a sleeping state as compared to a wakeful state. However, significantly for the instant disclosure, subjects who are experiencing an OSA episode have a dramatically lower EMG signal. This reduced EMG signal is evidence of a reduced tonal state of the protrusor muscles of the subjects experiencing OSA. FIG. 8 depicts similar data for comparison of the EMG signals during REM, non-REM, quiet wakefulness, and active wakefulness of subjects. This data confirms the top line of FIG. 7B that when asleep, the EMG signals are reduced, and as noted in FIG. 7B, that reduction is more pronounced and in subjects experiencing an OSA event.

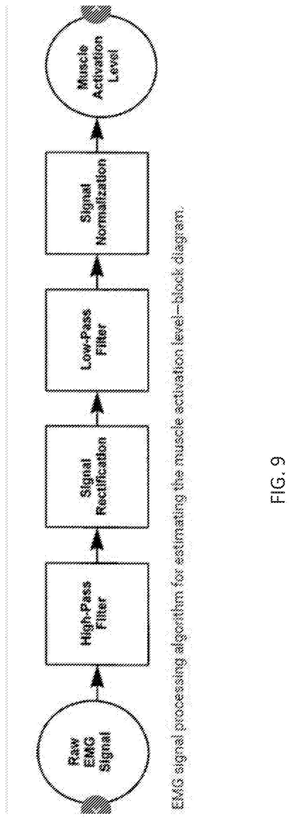

In accordance with one aspect of the disclosure, when a stimulation pulse is not being delivered by electrodes 30a-30d, the electrodes can be employed to detect the electrical potential of muscles. That is, the electrodes 30a-30d can detect the EMG signals that are being applied to the protrusor muscles by the patient's neural system. These signals can be communicated to the control circuit 80 for monitoring and application of rules in the software or firmware stored therein. In other examples, dedicated EMG sensing electrodes may be carried by housing 15 and/or lead body 22 and coupled to sensor 86 for EMG signal monitoring. EMG signal monitoring by control circuit 80 allows for detection of a low tonal state of the GG and/or GH muscles. With reference to FIG. 7A a low tonal state (i.e., low incidence of EMG signals) indicates both a likelihood of the patient being asleep and a susceptibility to upper airway collapse. Detection of low tonal state of the protrusor muscles may either alone or in combination with other sensor data, e.g., detection of the pose of the patient indicating that they are in a reclined position or the detection of a heart rate consistent with sleeping, be used to initiate therapy and prevent the onset of an OSA event. Thus, the EMG signals may be used by control circuit 80 to detect a sleep state and/or low tonal state of the protrusor muscles for use in controlling therapy delivery circuit 84 for delivering stimulation pulses to cause protrusion of the patient's tongue. As will be appreciated, in the detection of the EMG signals a variety of bandpass filtering, rectification, and normalization may be employed by the control circuit 80, or intervening hardware to produce a useable signal providing a clear indication of the state of the protrusor muscles. An example of such processing of the EMG signal is depicted in FIG. 9.

EMG monitoring may further be used in monitoring for fatigue of the stimulated GG and/or GH muscles. If fatigue of the muscles is detected, control circuit 80 may alter to control the duty cycle of electrical stimulation pulse trains delivered by therapy delivery circuit 84 to minimize or avoid fatigue and/or allow adequate fatigue recovery time between duty cycle on times. In this manner, Sensor 86 may be configured to produce a signal that is correlated to protrusor muscle tonal state for use by control circuit 80 for detecting a low tonal state predictive of upper airway obstruction, detecting protrusor muscle fatigue, and/or detecting a protruded state of tongue 40. Therapy delivery circuit 84 may be configured to respond to a detection of the protrusor muscle tonal state by control circuit 80 by adjusting one or more control parameters used to control stimulation pulse delivery.

As noted above, the EMG monitoring may not be the only signal employed by the pulse generator 12, and particularly the control circuit 80 in determining the level of wakefulness. As an example, sensor 86 may include an accelerometer that can provide an indication of motion of the patient. Further, where the accelerometer 86 is a three-axis accelerometer, a posture of the patient may be determined. Additionally, an accelerometer may be employed to detect snoring sounds and physical movements of the patient. Still further, a temperature sensor may be employed in which the diurnal temperature of a patient is measured and stored in memory as are sleeping temperatures. The sensor 86 may also be one or more accelerometers employed to detect the heartrate of a patient. In another example, sensor 86 may be an accelerometer employed to detect the rate of breathing or the volume of airflow, into or out of the patient. Volume of airflow may be determined by placing the accelerometer at a point on a patient's chest and comparing the travel of the accelerometer to previously observed lung volume data that has been correlated to the sensor 86 movement data. Breathing rate can be determined by simply monitoring the change in direction of the accelerometer.

Still further, the sensor 86 may be an implantable pulse-oximeter useable to measure the blood oxygen saturation levels. In one example the pulse-oximeter is a cuff placed substantially around a blood vessel and measuring the blood-oxygenation levels using a light source as is known in the art. As described herein, the sensor 86 may be one or several of the various types of sensors described herein.

The sensor 86 may be an ECG sensor. ECG is a recording of the electrical activity of the heart over a period of time. While an ECG typically employs sensors placed on the skin, an effective ECG can be employed in an implantable device wherein at least two electrodes separated by a distance (e.g., at least about 35mm) are employed to detect electrical changes caused by the cardiac depolarization and repolarization during each cardiac cycle.

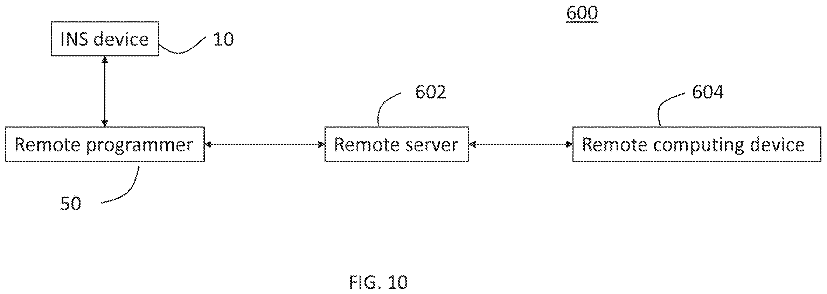

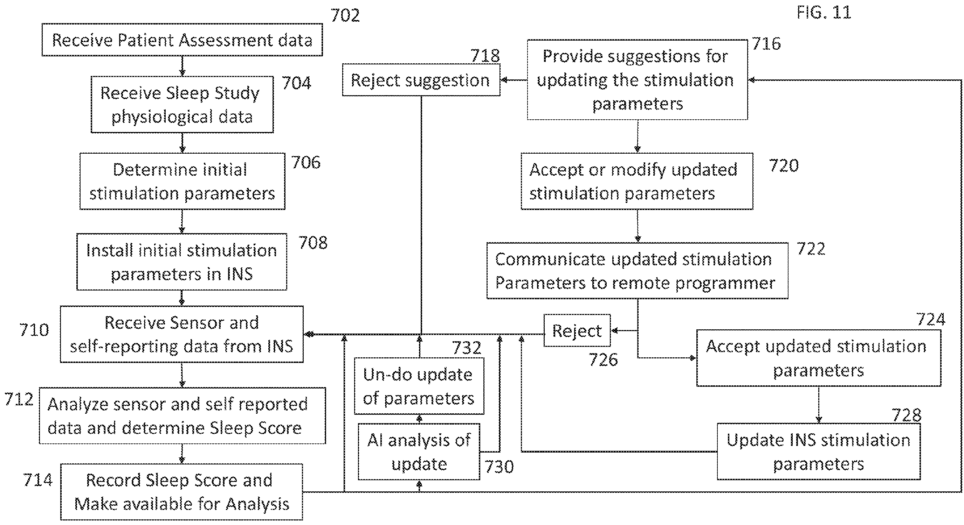

A further aspect of the disclosure is described in connection with FIGs. 10 and 11 in which a simplified diagram of an INS system is depicted, and a method of the systems operation are described. The system 600 includes an INS device 10, an external programmer 50, a server 602 in communication with the external programmer and a remote computer 604 in communication with the server 602. Prior to implantation of an INS 10, patients typically undergo a patient assessment (step 702) one or more analyses in conjunction with their doctor. During this analysis a variety of self-reported issues may be identified including daytime sleepiness, interrupted snoring, gasping, co-morbidities, etc. The data related to these issues may be stored on the server 602 as part of the patient electronic medical records (EMR) or as part of a specific OSA treatment and remediation file. The discussions with the medical provider may lead to an initial diagnosis of OSA. This initial diagnosis is typically confirmed through the use of one or more sleep studies of the patient. During the sleep study a wide variety of physiological data is gathered as well as some self-reported data. For example, the heart rate, blood oxygen saturation levels, temperature, an electroencephalogram (EEG), electrocardiogram (ECG), total sleep, quality of sleep, sleep efficiency, sleep stages, number of arousals (less than 15s), number of awakenings (greater than 15s), Apnea Hypopnea Index as well as others. These data may be recorded by remote computer 604, either directly or via additional hardware, and saved on the remote server 602 (step 704).

These collected data from the sleep study, along with the data collected by the medical provider may be used to generate an initial set of stimulation parameters (e.g., pulse width, frequency, amplitude, pairing of electrodes, etc.) for the INS 10 (step 706). This may be in part based on larger population studies to identify some aspects of more global therapy parameters. The initial stimulation parameters may be set either at remote computer 604 or directly at external programmer 50, and in either event may be saved a server 602 (e.g., a cloud computer storage device), for access by either device. And the external programmer 50 can be employed to install the initial stimulation parameters in the INS 10 (step 708). Often, the patient is permitted to utilize the INS 10 for a period of time, and a subsequent sleep study may be performed. From this second sleep study, the initial stimulation parameters may be altered, or additional surgery may be recommended to those who do not respond to stimulation therapy. Further sleep studies may be required periodically to adjust the stimulation parameter settings in an effort to improve the therapy of the individual patient.

In accordance with the present disclosure, the data collected from the sensor 86 may be combined with various self-reported data that a user may input via a user interface on the external programmer 50 and utilized to replace at least the second sleep study, and possibly the first as well. The external programmer 50, or another device in communication with the server 602, presents the patient with a user interface. The user interface may be presented to the user on a periodic basis including daily, weekly, bi-weekly, or monthly. In accordance, with the daily embodiment, the user interface may request that the patient input various self-reporting data. This can include nightly alcohol intake, smoking, stress, the time the patient went to bed, the patient's perception of the quality of the last night's sleep, tiredness, discomfort, pain or soreness of the protrusor muscles potentially caused by the stimulation, etc. Additionally, data from other appliances may also be reported. For example, may patients suffering from OSA also suffer from high blood pressure, and may be on a regimen of periodically testing their blood pressure. This blood pressure data may be self-reported via the user interface. Similarly, if the patient is a diabetic, they may need to test their blood sugar levels both before and after sleep. These data too may be self-reported via the user interface. In addition, the patient may be asked to answer the inquiries of the Eppsworth Sleepiness Scale (ESS). In one embodiment, the ESS inquiry may be requested of the patient at a different interval that the other data. In this way the ESS can be used as one gauge of the effectiveness of the therapy.

As noted above, the sensor 86 can provide a variety of data dependent upon how it is configured. As one example using the EMG data, a sleep start and end time may be determined. Using one or more accelerometers and a variety of bandpass filtering position, activity (arousals vs awakenings), sleep stages, respiration rate, and heart rate can be collected. This data can be reported to the control circuit 80 and stored in memory 82 at least temporarily. The external programmer 50 can be set to automatically interface with the INS 10 every day, or at another periodic interval. The external programmer 50 can then download the sensor data via the telemetry circuit and communicate the sensor data from the INS and self-reported data entered via the user-interface to the server 602 (step 710).

The server 602 may include thereon one or more software applications. One of these applications may review the data received from the external programmer 50 and assign a value to each datapoint received. These values can be analyzed, and a sleep score determined based on the received sensor and self-reported data (step 712). The sleep score provides an overall assessment of the patient's sleep that can be assessed by both the patient and the medical provider. As will be apparent some data points may be more important to assessing the overall sleep of the patient thus some form of scaling of the values may be required. The application will also be able to flag any relevant data significant to a poor sleep score. For example, if the patient reported several alcoholic drinks the evening before the data resulting in the poor sleep score was recorded, this might be a highly relevant factor, and indicate that the sleep score for that day is not an accurate indicator of the effectiveness of the current stimulation parameters.

Regardless, the sleep score may then be recorded as part of the patient's sleep record and the score reported to a medical provider via the remote computer 604 (step 714). This data may be viewed in a variety of ways to provide the medical provider an assessment of the current stimulation parameters. For example, the medical provider may view the daily results, an average over a period of time, a graphical representation of the sleep score or a percentage or rate of change (if any) from the preceding reporting period. By periodically assessing the effectiveness of the parameters and comparing the effectiveness to the additional self-reported data a multi-pronged analysis can be undertaken. In one example if the patient's data indicates that the therapy is effective in achieving quality sleep with few incidents of OSA, but the patient expresses a feeling of soreness or fatigue of the protrusor muscles, the stimulation parameters may be changed to increase the frequency of the switching between bi-polar pairs and to prolong the interval between any set of bi-polar pairs being stimulated. Alternatively, the amplitude of the signal may be reduced. Further, following further sampling of the data collected by the sensor 86 and self-reported by the patient via the user interface, if the first of these is not effective, the second may be attempted. In this way, the medical provider is able to proceed in a stepwise fashion of altering the stimulation parameters, make adjustments, and observing the results of those adjustments while considering not just the self-reported data. This collection of data and reporting of a sleep score (steps 710-714) may be iterative repeated prior to advancing to the next step. One of skill in the art will recognize that the remote computer may in fact be an external programmer 50 configured for physician or medical care provider's use.

In a further aspect of the disclosure, the server 602 may collect or be in communication one or more further servers receiving similar data from other patients. The entirety of the collected data may then be analyzed by one or more neural networks to assess the combined data and to identify patterns within the data to provide a global assessment of stimulation parameters and effectiveness of the stimulation pattens when applied across a wide array of patients. Some of these patients will have similar comorbidities, and others will not. By further assessment of the data the neural network can seek out similar groups of patients and provide refined initial stimulation parameters for the subgroup based on these similarities (e.g., age, demographics, weight, heart disease, blood pressure, etc.). The neural network may also be employed to assess an individual patient to provide individualized guidance on updating stimulation parameters. In a similar fashion, the server may include one or more applications employing fuzzy logic to analyze the data from both an individual and from the broader community of patients to provide suggestions for updating the stimulation parameters (step 716). In both the use of neural networks and fuzzy logic the application on the server 602 may present the medical provider with the option to reject the suggested updated stimulation parameters (step 718) or to accept or modify the suggested updated stimulation parameters (step 720). As will be appreciated, the medical provider may forgo the use of either the neural network or fuzzy logic and update or modify the stimulation parameters. Once the updated stimulation parameters are accepted/modified by the medical provider the updated stimulation parameters are communicated to the external programmer 50 (step 722). Once received at the external programmer 50, the patient again may have the option to accept the updated stimulation parameters (step 724) or reject the updated stimulation parameters (step 726). If accepted by the patient the external programmer 50 can update the stimulation parameters on the INS (step 728). If at step 718 or 726 the updated stimulation parameters are rejected, the method simply returns to step 710. Similarly, after updating the stimulation parameters on the INS 10, the process similarly returns to step 710.

These updated stimulation parameters may be stored on the server 602 until the next communication with the remote programmer 50, at which time the improved stimulation parameters may be downloaded to the external programmer 50. During the next collection of data from the INS 10, the external programmer 50 may then download the updated stimulation parameters to the INS 10. In this way, the stimulation parameters of the INS updated, and the patient's sleep score improved. As would be expected the user interface on the external programmer 50 would indicate to the patient that the new stimulation parameters are ready for installing on the INS, and it is at this point that steps 726 and 724 may be performed.

A further aspect of the present disclosure is the presence of an artificial intelligence (AI) within the external programmer 50. The AI may have a limited mandate for purposes of safety of the patient to limit the number of successive nights resulting in a poor sleep score. In one aspect of the disclosure, following the update of the stimulation parameters and receiving the data from the following night's sleep, the AI could analyze the data from sensor 86 and the self-reported data and make an immediate assessment regarding the sleep score (step 730). If the sleep score is bad the user interface may present the patient with the ability to revert to the prior stimulation parameters until they can interface with their medical provider regarding the prior bad night's sleep (step 732). Of course, the AI may require more than a single night's data to identify the issue or have sufficient data to raise concerns with the patient. Further, the AI may be enabled to communicate a request for intervention directly to the medical provider via the server 602 and remote computer 604.

In this way real actionable feedback on the effectiveness of stimulation parameters is provided to both the medical provider and to the patient. A continuum of care and assessment of the patient's experience with the INS device is enabled so that adverse results from therapy can be rectified and behavioral modifications can be suggested to the patient based on their self-reported data.

It will be appreciated by those of skill in the art that one or more of the calculations, assessments, and user interfaces described herein above with respect to the server 602 and the remote computer 604 may also be performed directly at the external programmer 50. In some embodiments this may provide for near instant feedback to the patient regarding a prior night's sleep via a user interface on the external programmer. In other embodiments, where the external programmer 50 is of a type typically used by the physician during an office visit, the capabilities and functions of the external programmer 50 can be more robust and potentially even eliminate or at least reduce the use of the server 602 and remote computer 604. As a further, example, in applications employing an AI, the AI may be trained to perform all of the assessments and analyses of the server and offer up the suggestions for modification of the stimulation parameters to the patient or care provider. allowing a greater depth of understanding of the therapy, efficacy, and assessment of possible changes, without necessarily requiring access to the data stored on the server 602 or the applications operating thereon. And in yet a further example, the AI on the external programmer 50 may assess the data received from the INS 10 and make adjustments to the stimulation parameters either autonomously or present them to the patient for acceptance. As will be appreciated, these updates may be bounded to prevent large changes in the stimulation parameters from occurring without intervention from a medical provider.

It should be understood that, depending on the example, certain acts or events of any of the methods described herein can be performed in a different sequence, may be added, merged, or left out altogether (e.g., not all described acts or events are necessary for the practice of the method). Moreover, in certain examples, acts or events may be performed concurrently, e.g., through multi-threaded processing, interrupt processing, or multiple processors, rather than sequentially. In addition, while certain aspects of this disclosure are described as being performed by a single module or unit for purposes of clarity, it should be understood that the techniques of this disclosure may be performed by a combination of units or modules associated with, for example, a medical device.

In one or more examples, the functions described may be implemented in hardware, software, firmware, or any combination thereof. If implemented in software, the functions may be stored as one or more instructions or code on a computer-readable medium and executed by a hardware-based processing unit. Computer-readable media may include computer-readable storage media, which corresponds to a tangible medium such as data storage media (e.g., RAM, ROM, EEPROM, flash memory, or any other medium that can be used to store desired program code in the form of instructions or data structures and that can be accessed by a computer).

Instructions may be executed by one or more processors, such as one or more digital signal processors (DSPs), general purpose microprocessors, application specific integrated circuits (ASICs), field programmable logic arrays (FPGAs), or other equivalent integrated or discrete logic circuitry. Accordingly, the term "processor," as used herein may refer to any of the foregoing structure or any other structure suitable for implementation of the techniques described herein. Also, the techniques could be fully implemented in one or more circuits or logic elements.

Thus, an implantable medical device system has been presented in the foregoing description with reference to specific examples. It is to be understood that various aspects disclosed herein may be combined in different combinations than the specific combinations presented in the accompanying drawings. It is appreciated that various modifications to the referenced examples may be made. The scope of the invention is defined by the claims.

## Claims

1. An implantable neurostimulator INS, comprising:
an electrical lead (20) having formed thereon at least a pair of bipolar intramuscular ring electrodes (30), wherein the electrical lead is configured for placement of the ring electrodes within protrusor muscles of a patient in an area of a medial branch of a hypoglossal nerve, that includes motor points of the one or more protrusor muscles of the patient, wherein the motor points are where each nerve axon of the hypoglossal nerve terminates in the muscle, and wherein the ring electrodes are configured to receive electromyography. EMG resulting from signals applied to the protrusor muscles by the patient's neural system from the intramuscular electrodes;
a pulse generator (12) electrically connected to the electrical lead and configured to deliver electrical energy to the ring electrodes to activate the protrusor muscles to move the tongue of the patient forward, the pulse generator having therein a sensor (86) and a control circuit (80),
wherein the sensor and control circuit are configured to receive the EMG signals sensed with the ring electrodes from the one or more protrusor muscles and determine a tonal state of the protrusor muscles in which the lead is placed based on the EMG signals; and wherein the pulse generator (12) includes a therapy delivery circuit (84) and the control circuit (80) is in electrical communication with the therapy delivery circuit (84) and causes the therapy delivery circuit to deliver electrical energy to the ring electrodes upon a determination that the EMG signal is below a threshold value.

2. The implantable neurostimulator of claim 1, wherein the control circuit is configured to cause the therapy delivery circuit to deliver electrical energy to the intramuscular ring electrodes upon a determination that the EMG signal is below a threshold value and a determination that a heart rate detected by the sensor is below a heart rate threshold; or wherein the control circuit is configured to cause the therapy delivery circuit to deliver electrical energy to the intramuscular ring electrodes upon a determination that the EMG signal is below an EMG threshold value and a motion sensor determines that the INS is not moving, or wherein the control circuit is configured to cause the therapy delivery circuit to deliver electrical energy to the intramuscular electrodes upon a determination that the EMG signal is below an EMG threshold value and an acoustic sensor detects sounds consistent with snoring; or wherein the control circuit is configured to cause the therapy delivery circuit to deliver electrical energy to the intramuscular electrodes upon a determination that the EMG signal is below an EMG threshold value and a temperature sensor detects a body temperature consistent with sleeping; or wherein the control circuit is configured to cause the therapy delivery circuit to deliver electrical energy to the intramuscular electrodes upon a determination that the EMG signal is below an EMG threshold value and a breathing rate sensor detects a breathing rate consistent with sleeping.

3. A system comprising:
an implantable neurostimulators, INS, as claimed in any preceding claim, the pulse generator further including a memory (82) and a telemetry circuit (88);
an external programmer (50) in communication with the INS via the telemetry circuit;
a server in communication with the external programmer and including thereon an application configured to receive sensor data from the INS from the external programmer and assess a quality of the sleep of a patient in which the INS is implanted based on the received sensor data; and
a remote computer in communication with the server and configured to present an assessment of the quality of sleep of the patient.

4. The system of claim 3, further comprising a user interface presented on the external programmer and configured to receive self-reported data entered by the patient.

5. The system of claim 4, wherein the application is further configured to assess the quality of the sleep of a patient in which the INS is implanted based on the received sensor data and the self-reported data, and optionally wherein the assessment of the quality of sleep is presented in the form of a sleep score.

6. The system of claim 3, 4 or 5, wherein the application is configured to assess the quality of the sleep of a patient in which the INS is implanted based on the received sensor data and self-reported data entered via a user interface on the external programmer and to determine a set of suggested updated stimulation parameters for the INS.

7. The system of claim 6, wherein the received sensor data includes one or more of a tonal state of protrusor muscles, heartrate, blood pressure, blood oxygen saturation, patient temperature, arousals, awakenings, and electromyography data.

8. The system of claim 6 or 7, wherein the updated stimulation parameters are available for review, acceptance, modification, or rejection on the remote computer.

9. The system of claim 8, wherein upon acceptance or modification of the updated stimulation parameters, the updated stimulation parameters are transmitted to the external programmer.

10. The system of claim 9, wherein the external programmer is configured to transmit the updated stimulation parameters to the INS.

## Patentansprüche

1. Implantierbarer Neurostimulator, INS, umfassend:
eine elektrische Leitung (20), die mindestens ein Paar von bipolaren intramuskulären Ringelektroden (30) darauf ausgebildet aufweist, wobei die elektrische Leitung für eine Platzierung der Ringelektroden innerhalb der Protrusormuskeln eines Patienten in einem Bereich eines medialen Astes eines Nervus hypoglossus konfiguriert ist, der motorische Punkte der einen oder mehrerer Protrusormuskeln des Patienten einschließt, wobei sich die motorischen Punkte dort befinden, wo jedes Nervenaxon des Nervus hypoglossus in dem Muskel endet, und wobei die Ringelektroden konfiguriert sind, um Elektromyographie, EMG, zu empfangen, die aus Signalen resultiert, die durch das Nervensystem eines Patienten von den intramuskulären Elektroden an die Protrusormuskeln angelegt werden;
einen Impulsgenerator (12), der mit der elektrischen Leitung elektrisch verbunden und konfiguriert ist, um elektrische Energie an die Ringelektroden zu liefern, um die Protrusormuskeln zu aktivieren und die Zunge des Patienten nach vorne zu bewegen, wobei der Impulsgenerator einen Sensor (86) und eine Steuerschaltung (80) darin aufweist,
wobei der Sensor und die Steuerschaltung konfiguriert sind, um die EMG-Signale zu empfangen, die mit den Ringelektroden von dem einem oder den mehreren Protrusormuskeln erfasst werden, und basierend auf den EMG-Signalen einen tonalen Zustand der Protrusormuskeln zu bestimmen, in dem die Leitung platziert ist; und wobei der Impulsgenerator (12) eine Therapieabgabeschaltung (84) einschließt und die Steuerschaltung (80) in elektrischer Kommunikation mit der Therapieabgabeschaltung (84) steht und die Therapieabgabeschaltung veranlasst, elektrische Energie an die Ringelektroden bei einer Bestimmung abzugeben, dass das EMG-Signal unter einem Schwellenwert liegt.

2. Implantierbarer Neurostimulator nach Anspruch 1, wobei die Steuerschaltung konfiguriert ist, um die Therapieabgabeschaltung zu veranlassen, elektrische Energie an die intramuskulären Ringelektroden bei einer Bestimmung, dass das EMG-Signal unter einem Schwellenwert liegt, und einer Bestimmung abzugeben, dass eine Herzfrequenz, die durch den Sensor erkannt wird, unter einem Herzfrequenzschwellenwert liegt; oder wobei die Steuerschaltung konfiguriert ist, um die Therapieabgabeschaltung zu veranlassen, elektrische Energie an die intramuskulären Ringelektroden bei einer Bestimmung abzugeben dass das EMG-Signal unter einem EMG-Schwellenwert liegt und ein Bewegungssensor bestimmt, dass sich der INS nicht bewegt, oder wobei die Steuerschaltung konfiguriert ist, um die Therapieabgabeschaltung zu veranlassen, elektrische Energie an die intramuskulären Elektroden bei einer Bestimmung abzugeben, dass das EMG-Signal unter einem EMG-Schwellenwert liegt und ein Akustiksensor Geräusche erkennt, die mit Schnarchen übereinstimmen; oder wobei die Steuerschaltung konfiguriert ist, um die Therapieabgabeschaltung zu veranlassen, elektrische Energie an die intramuskulären Elektroden bei einer Bestimmung abzugeben, dass das EMG-Signal unter einem EMG-Schwellenwert liegt und ein Temperatursensor eine Körpertemperatur erkennt, die mit Schlafen übereinstimmt; oder wobei die Steuerschaltung konfiguriert ist, um die Therapieabgabeschaltung zu veranlassen, elektrische Energie an die intramuskulären Elektroden bei einer Bestimmung abzugeben, dass das EMG-Signal unter einem EMG-Schwellenwert liegt und ein Atemfrequenzsensor eine Atemfrequenz erkennt, die mit Schlafen übereinstimmt.

3. System, umfassend:
einen implantierbaren Neurostimulator, INS, nach einem der vorstehenden Ansprüche, wobei der Impulsgenerator ferner einen Speicher (82) und eine Telemetrieschaltung (88) einschließt;
einen externen Programmierer (50), der über die Telemetrieschaltung mit dem INS in Kommunikation steht;
einen Server, der mit dem externen Programmierer in Kommunikation steht und eine Anwendung darauf einschließt, die konfiguriert ist, um Sensordaten von dem INS von dem externen Programmierer zu empfangen und die Schlafqualität eines Patienten, in den der INS implantiert ist, basierend auf den empfangenen Sensordaten zu beurteilen; und
ein entfernten Computer, der mit dem Server in Kommunikation steht und konfiguriert ist, um eine Bewertung der Schlafqualität des Patienten vorzulegen.

4. System nach Anspruch 3, ferner umfassend eine Benutzerschnittstelle, die auf dem externen Programmierer vorgelegt wird und konfiguriert ist, um Selbstberichtsdaten zu empfangen, die durch den Patienten eingegeben werden.

5. System nach Anspruch 4, wobei die Anwendung ferner konfiguriert ist, um die Schlafqualität eines Patienten, in den der INS implantiert ist, basierend auf den empfangenen Sensordaten und den selbst gemeldeten Daten zu beurteilen, und optional wobei die Beurteilung der Schlafqualität in Form einer Schlafbewertung vorgelegt wird.

6. System nach Anspruch 3, 4 oder 5, wobei die Anwendung konfiguriert ist, um die Schlafqualität eines Patienten, in den der INS implantiert ist, basierend auf den empfangenen Sensordaten und den selbst gemeldeten Daten zu beurteilen, die über eine Benutzerschnittstelle auf den externen Programmierer eingegeben werden, und um einen Satz vorgeschlagener aktualisierter Stimulationsparameter für den INS zu bestimmen.

7. System nach Anspruch 6, wobei die empfangenen Sensordaten einen oder mehrere von einem tonalen Zustand der Protrusormuskeln, einer Herzfrequenz, einem Blutdruck, einer Blutsauerstoffsättigung, einer Patiententemperatur, Erregungszuständen, einem Aufwachen und Elektromyographiedaten einschließen.

8. System nach Anspruch 6 oder 7, wobei die aktualisierten Stimulationsparameter auf dem entfernten Computer für eine Überprüfung, eine Annahme, eine Änderung oder eine Ablehnung verfügbar sind.

9. System nach Anspruch 8, wobei, nach der Annahme oder der Änderung der aktualisierten Stimulationsparameter, die aktualisierten Stimulationsparameter an den externen Programmierer übertragen werden.

10. System nach Anspruch 9, wobei der externe Programmierer konfiguriert ist, um die aktualisierten Stimulationsparameter an den INS zu übertragen.

## Revendications

1. Neurostimulateur implantable, INS, comprenant :
une sonde électrique (20) ayant au moins une paire d'électrodes annulaires intramusculaires bipolaires (30) formée sur celle-ci, dans lequel la sonde électrique est configuré pour placer les électrodes annulaires dans les muscles protrusor d'un patient dans une zone d'une branche médiane d'un nerf hypoglosse, qui comporte des points moteurs du ou des muscles protrusor du patient, dans lequel les points moteurs se situent là où chaque axone du nerf hypoglosse se termine dans le muscle, et dans lequel les électrodes annulaires sont configurées pour recevoir l'électromyographie, EMG, résultant des signaux appliqués aux muscles protrusor par le système neuronal du patient à partir des électrodes intramusculaires ;
un générateur d'impulsions (12) connecté électriquement à la sonde électrique et configuré pour fournir de l'énergie électrique aux électrodes annulaires afin d'activer les muscles protrusor pour déplacer la langue du patient vers l'avant, le générateur d'impulsions ayant un capteur (86) et un circuit de commande (80) en son sein,
dans lequel le capteur et le circuit de commande sont configurés pour recevoir les signaux EMG captés par les électrodes annulaires à partir du ou des muscles protrusor et pour déterminer un état tonal des muscles protrusor dans lesquels la sonde est placée, sur la base des signaux EMG ; et dans lequel le générateur d'impulsions (12) comporte un circuit d'administration de thérapie (84) et le circuit de commande (80) est en communication électrique avec le circuit d'administration de thérapie (84) et amène le circuit d'administration de thérapie à fournir de l'énergie électrique aux électrodes annulaires après une détermination que le signal EMG est inférieur à une valeur seuil.

2. Neurostimulateur implantable selon la revendication 1, dans lequel le circuit de commande est configuré pour amener le circuit d'administration de thérapie à fournir de l'énergie électrique aux électrodes annulaires intramusculaires après une détermination que le signal EMG est inférieur à une valeur seuil et une détermination qu'une fréquence cardiaque détectée par le capteur est inférieure à un seuil de fréquence cardiaque ; ou dans lequel le circuit de commande est configuré pour amener le circuit d'administration de thérapie à fournir de l'énergie électrique aux électrodes annulaires intramusculaires après une détermination que le signal EMG est inférieur à une valeur seuil EMG et qu'un capteur de mouvement détermine que l'INS ne se déplace pas, ou dans lequel le circuit de commande est configuré pour amener le circuit d'administration de thérapie à fournir de l'énergie électrique aux électrodes intramusculaires lorsqu'il est déterminé que le signal EMG est inférieur à une valeur seuil EMG et qu'un capteur acoustique détecte des bruits correspondant à des ronflements ; ou dans lequel le circuit de commande est configuré pour amener le circuit d'administration de thérapie à fournir de l'énergie électrique aux électrodes intramusculaires après une détermination que le signal EMG est inférieur à une valeur seuil EMG et qu'un capteur de température détecte une température corporelle correspondant au sommeil ; ou dans lequel le circuit de commande est configuré pour amener le circuit d'administration de thérapie à fournir de l'énergie électrique aux électrodes intramusculaires après une détermination que le signal EMG est inférieur à une valeur seuil EMG et qu'un capteur de fréquence respiratoire détecte une fréquence respiratoire correspondant au sommeil.

3. Système comprenant :
un neurostimulateur implantable, INS, selon l'une quelconque revendication précédente, le générateur d'impulsions comportant en outre une mémoire (82) et un circuit de télémétrie (88) ;
un programmateur externe (50) en communication avec l'INS par l'intermédiaire du circuit de télémétrie ;
un serveur en communication avec le programmateur externe et comportant sur celui-ci une application configurée pour recevoir des données de capteur de l'INS à partir du programmateur externe et évalue la qualité du sommeil d'un patient chez qui l'INS est implanté, sur la base des données de capteur reçues ; et
un ordinateur distant en communication avec le serveur et configuré pour présenter une évaluation de la qualité du sommeil du patient.

4. Système selon la revendication 3, comprend en outre une interface utilisateur présentée sur le programmateur externe et configurée pour recevoir les données autodéclarées entrées par le patient.

5. Système selon la revendication 4, dans lequel l'application est en outre configurée pour évaluer la qualité du sommeil d'un patient chez qui l'INS est implanté, sur la base des données de capteur reçues et des données autodéclarées, et éventuellement dans lequel l'évaluation de la qualité du sommeil est présentée sous la forme d'un score de sommeil.

6. Système selon la revendication 3, 4 ou 5, dans lequel l'application est configurée pour évaluer la qualité du sommeil d'un patient chez qui l'INS est implanté, sur la base des données de capteur reçues et des données autodéclarées entrées par l'intermédiaire d'une interface utilisateur sur le programmateur externe, et pour déterminer un ensemble de paramètres de stimulation actualisés suggérés pour l'INS.

7. Système selon la revendication 6, dans lequel les données de capteur reçues comportent l'un ou plusieurs parmi l'état tonal des muscles protrusor, la fréquence cardiaque, la pression artérielle, la saturation en oxygène du sang, la température du patient, les éveils, les réveils et les données d'électromyographie.

8. Système selon la revendication 6 ou 7, dans lequel les paramètres de stimulation mis à jour sont disponibles pour examen, acceptation, modification ou rejet sur l'ordinateur distant.

9. Système selon la revendication 8, dans lequel, après acceptation ou modification des paramètres de stimulation mis à jour, les paramètres de stimulation mis à jour sont transmis au programmateur externe.

10. Système selon la revendication 9, dans lequel le programmateur externe est configuré pour transmettre les paramètres de stimulation mis à jour à l'INS.
